# EUROPEAN PATENT APPLICATION

(11) **EP 4 541 266 A1**
(43) Date of publication of application: **23.04.2025**
(21) Application number: 24207541.4
(22) Date of filing: 18.10.2024
(51) Int. Cl.: A61B 5/00, A61B 5/145

(54) **BLOOD GLUCOSE MEASUREMENT DEVICE AND METHOD FOR MANAGING BLOOD GLUCOSE MEASUREMENT DATA USING THE SAME**

(30) Priority: 19.10.2023 KR 20230140129
(71) Applicant: i-Sens, Inc., Seoul 06646 (KR)
(72) Inventor: KIM, Dong Su, 14325 Gwangmyeong-si, Gyeonggi-do (KR); KIM, Hyoung Soo, 01429 Seoul (KR); YOU, Choong Beom, 02849 Seoul (KR); LEE, Jeong Jik, 26494 Wonju-si, Gangwon-do (KR); LEE, Hye Jin, 16330 Suwon-si, Gyeonggi-do (KR)
(74) Representative: Isarpatent

(57) **Abstract**

A blood glucose measurement device according to an embodiment of the present invention comprises: a measurement unit that is partially inserted into the body of a subject and generates a blood glucose measurement signal by measuring a blood glucose concentration of the subject; a control unit that generates blood glucose measurement data by converting the blood glucose measurement signal into digital data; a storage unit that includes a plurality of storage areas and stores the blood glucose measurement data in the plurality of storage areas; and a communication unit that transmits the blood glucose measurement data stored in at least one of the plurality of storage areas to an external device, wherein the control unit determines the validity of the blood glucose measurement data stored in the plurality of storage areas, and controls the communication unit to transmit the blood glucose measurement data stored in at least one of the plurality of storage areas to the external device, based on a result of the determination.

## Description

### CROSS REFERENCE TO RELATED APPLICATION

The present application claims the priority of Korean Patent Application No. 10-2023-0140129 filed on 19 10, 2023, the entire contents of which is incorporated herein for all purposes by this reference.

### BACKGROUND OF THE INVENTION

### Field of the Invention

The embodiment relates to a blood glucose measurement device capable of continuously measuring a blood glucose level of a subject and a method for managing blood glucose measurement data of the blood glucose measurement device.

### Description of the Related Art

Diabetes is a chronic disease that occurs frequently in modern people, and in Korea, it affects more than 2 million people, which is 5% of the total population.

Diabetes occurs when the insulin produced by a pancreas is absolutely or relatively insufficient due to various causes such as obesity, stress, bad eating habits, and congenital heredity, so that a glucose content in the blood increases significantly by failing to correct the blood glucose balance.

Blood usually contains a certain concentration of glucose, and a tissue cell obtains energy from glucose.

However, if glucose increases more than necessary, it is not stored properly in a liver, muscle, or a fat cell, and is accumulated in the blood. As a result, a diabetic patient maintains much higher blood glucose level than normal people, and as the excessive blood glucose passes through the tissues as it is and is excreted in the urine, the glucose that is absolutely necessary for each tissue of the body becomes insufficient to cause abnormalities in each tissue of the body.

Diabetes is characterized by having almost no symptoms in the early stages. However, as the disease progresses, it causes symptoms specific to diabetes such as large beverage consumption, polyphagia, polyuria, weight loss, general malaise, itchy skin, and long-lasting wounds on hands and feet that do not heal, and as the disease progresses further, complications that progress to visual impairment, hypertension, kidney disease, stroke, periodontal disease, muscle cramps and neuralgia, gangrene, etc. are caused.

In order to diagnose such diabetes and manage it so as not to progress to complications, systematic blood glucose measurement and treatment must be performed in parallel.

Since diabetes requires constant blood glucose measurement for management, the demand for blood glucose measurement devices is steadily increasing. Various studies have confirmed that if diabetic patients strictly control their blood glucose, the occurrence of diabetic complications is significantly reduced. Accordingly, it is very important for diabetic patients to regularly measure their blood glucose level to control their blood glucose.

A blood collection-typed blood glucose meter (finger prick method) is generally used to manage blood glucose in diabetic patients. Although this blood collection-typed blood glucose meter helps diabetic patients manage blood glucose, it is difficult to accurately grasp blood glucose levels that change frequently because only the results appear at the time of measurement. In addition, the blood collection-typed blood glucose meter requires blood to be drawn multiple times a day to measure blood glucose level, which poses a significant burden to diabetic patients.

Diabetic patients generally travel between hyperglycemia and hypoglycemia, and an emergency occurs in hypoglycemia. Hypoglycemia occurs when glucose levels do not last long, and may lead to loss of consciousness or death in the worst case.

Therefore, it is very important for diabetic patients to immediately detect hypoglycemia. However, the blood collection-typed blood glucose meter that measures blood glucose intermittently has clear limitations.

In order to overcome the limitations of the blood collection-typed blood glucose meter, a continuous glucose monitoring system (CGMS) that is inserted into the body and measures blood glucose at intervals of several minutes has been developed, and this system can be used to easily manage diabetes patients and respond to emergencies.

The continuous glucose monitoring system is configured to include a transmitter that is attached to a user's body part to measure blood glucose by extracting body fluid, and a communication terminal that outputs the transmitted blood glucose level. The sensor transmitter generates blood glucose information by measuring the user's blood glucose for a certain period of time, for example, for approximately 15 days, while the sensor is inserted into the body. The sensor transmitter periodically generates blood glucose information, and the communication terminal is installed with a blood glucose management application to periodically receive blood glucose information from the transmitter and output the received blood glucose information so that the user can check it. In this case, the transmitter stores the measured blood glucose information in a memory before generating the blood glucose information and transmitting it to the communication terminal.

However, the small embedded device such as the transmitter may experience temporary power supply problems due to external impact or electromagnetic waves. If such problems occur during storing data in a non-volatile memory of the transmitter, the data may not be stored normally. When the stored data is read to control the operation of the transmitter or transmit it to other device, if the data is not completely stored data, it may cause serious problems.

Background art of the present invention includes Japanese Patent Laid-open Publication No. 2011-062335 and U.S. Patent Application Publication No. US2010/0168537.

### SUMMARY OF THE INVENTION

The embodiment provides a blood glucose measurement device for providing accuracy and completeness to blood glucose measurement data of a subject, and a method for managing the blood glucose measurement data using the same.

The challenge which is intended to be solved by the embodiment is not limited thereto, and may also include the purpose or effect that can be identified from the solution or embodiment of the challenge to be described below.

A blood glucose measurement device according to an embodiment of the present invention comprises: a measurement unit that is partially inserted into the body of a subject and generates a blood glucose measurement signal by measuring a blood glucose concentration of the subject; a control unit that generates blood glucose measurement data by converting the blood glucose measurement signal into digital data; a storage unit that includes a plurality of storage areas and stores the blood glucose measurement data in the plurality of storage areas; and a communication unit that transmits the blood glucose measurement data stored in at least one of the plurality of storage areas to an external device,
wherein the control unit determines the validity of the blood glucose measurement data stored in the plurality of storage areas, and controls the communication unit to transmit the blood glucose measurement data stored in at least one of the plurality of storage areas to the external device, based on a result of the determination.

The storage unit stores the blood glucose measurement data in a first storage area included in the plurality of storage areas, and stores the blood glucose measurement data in a second storage area included in the plurality of storage areas, wherein the blood glucose measurement data stored in the first storage area may match with the blood glucose measurement data stored in the second storage area.

The communication unit may transmit the blood glucose measurement data stored in the first storage area to the external device if the blood glucose measurement data stored in the first storage area is determined to be valid; and may transmit the blood glucose measurement data stored in the second storage area to the external device if the blood glucose measurement data stored in the first storage area is determined to be invalid.

The storage unit may store the blood glucose measurement data in the first storage area included in the plurality of storage areas; and may store the blood glucose measurement data in the second storage area included in the plurality of storage areas if a preset error event is determined to have occurred in the first storage area.

The storage unit may store the blood glucose measurement data by crossing the first storage area and the second storage area included in the plurality of storage areas.

The storage unit may store first blood glucose measurement data in a first storage space of the first storage area; and store second blood glucose measurement data in a second storage space of the second storage area, wherein the first storage space and the second storage space of the first storage area correspond to the first storage space and the second storage space of the second storage area, respectively, and the first storage space and the second storage space may be continuous with each other.

The storage unit may store the first blood glucose measurement data in the first storage space of the first storage area; and store the second blood glucose measurement data in the first storage space of the second storage area, wherein the first storage space of the first storage area may correspond to the first storage space of the second storage area.

The control unit may check the validity of the blood glucose measurement data stored in the first storage area and the second storage area; and the communication unit may include a step of transmitting blood glucose measurement data determined to be valid among the blood glucose measurement data stored in the first storage area and the second storage area to the external device.

A method for managing blood glucose measurement data using a blood glucose measurement device that continuously measures blood glucose of a subject according to an embodiment of the present invention comprises the steps of: generating a blood glucose measurement signal by measuring blood glucose of the subject; converting the blood glucose measurement signal into blood glucose measurement data that is a digital signal; storing the blood glucose measurement data in a plurality of storage areas; determining the validity of the blood glucose measurement data stored in the plurality of storage areas; and transmitting the blood glucose measurement data stored in at least one of the plurality of storage areas to an external device, based on a result of the determination.

The step of storing the blood glucose measurement data may include the steps of: storing the blood glucose measurement data in a first storage area included in the plurality of storage areas; and storing the blood glucose measurement data in a second storage area included in the plurality of storage areas, wherein the blood glucose measurement data stored in the first storage area may match the blood glucose measurement data stored in the second storage area.

The step of transmitting the blood glucose measurement data stored in at least one of the plurality of storage areas to the external device may include the steps of: transmitting the blood glucose measurement data stored in the first storage area to the external device if the blood glucose measurement data stored in the first storage area is determined to be valid; and transmitting the blood glucose measurement data stored in the second storage area to the external device if the blood glucose measurement data stored in the first storage area is determined to be invalid.

The step of storing the blood glucose measurement data may include the steps of: storing the blood glucose measurement data in a first storage area included in the plurality of storage areas; determining whether a preset error event has occurred in the first storage area; and storing the blood glucose measurement data in a second storage area included in the plurality of storage areas if it is determined that the error event has occurred.

The step of storing the blood glucose measurement data may store the blood glucose measurement data by crossing the first storage area and the second storage area included in the plurality of storage areas.

The step of storing the blood glucose measurement data includes the step of: storing first blood glucose measurement data in a first storage space of the first storage area; and storing second blood glucose measurement data in a second storage space of the second storage area, wherein the first storage space and the second storage space of the first storage area correspond to the first storage space and the second storage space of the second storage area, respectively, and the first storage space and the second storage space may be continuous with each other.

The step of storing the blood glucose measurement data includes the steps of: storing the first blood glucose measurement data in the first storage space of the first storage area; and storing the second blood glucose measurement data in the first storage space of the second storage area, wherein the first storage space of the first storage area may correspond to the first storage space of the second storage area.

The step of transmitting the blood glucose measurement data stored in at least one of the plurality of storage areas to the external device includes the steps of: checking the validity of the blood glucose measurement data stored in the first storage area and the second storage area; and transmitting blood glucose measurement data determined to be valid among the blood glucose measurement data stored in the first storage area and the second storage area to the external device.

According to an embodiment, the present invention can provide high accuracy and completeness of the blood glucose measurement data, and a minimum loss rate of data, since the transmitter stores the blood glucose measurement data by overlapping and/or crossing a plurality of storage areas so that even if an error occurs in one of the storage areas later, data from the storage area where no error occurs can be used.

The various and beneficial advantages and effects of the present invention are not limited to the above-described contents, and will be able to be more easily understood in the course of disclosing specific embodiments of the present invention.

### BRIEF DESCRIPTION OF THE DRAWINGS

FIG. 1 is a schematic diagram illustrating a continuous glucose monitoring system according to an embodiment of the present invention.
FIG. 2 is a diagram for explaining configurations of a blood glucose measurement device according to an embodiment of the present invention.
FIG. 3 is a diagram for explaining the first embodiment of a blood glucose data storage process according to an embodiment of the present invention.
FIG. 4 is a diagram for explaining the second embodiment of a blood glucose data storage process according to an embodiment of the present invention.
FIG. 5 is a diagram for explaining the third embodiment of a blood glucose data storage process according to an embodiment of the present invention.
FIG. 6 is a diagram for explaining the fourth embodiment of a blood glucose data storage process according to an embodiment of the present invention.
FIG. 7 is a diagram for explaining the first embodiment of a blood glucose data storage process according to an embodiment of the present invention.
FIG. 8 is the first embodiment for explaining a blood glucose measurement data transmission process according to an embodiment of the present invention.
FIG. 9 is the second embodiment for explaining a blood glucose measurement data transmission process according to an embodiment of the present invention.
FIG. 10 is the third embodiment for explaining a blood glucose measurement data transmission process according to an embodiment of the present invention.
FIG. 11 is the fourth embodiment for explaining a blood glucose measurement data transmission process according to an embodiment of the present invention.
FIG. 12 is the fifth embodiment for explaining a blood glucose measurement data transmission process according to an embodiment of the present invention.
FIG. 13 is a flowchart illustrating a method for managing blood glucose measurement data according to an embodiment of the present invention.
FIG. 14 is an embodiment showing step S1330 of FIG. 13 in detail.
FIG. 15 is an embodiment showing step S1330 of FIG. 13 in detail.
FIG. 16 is an embodiment showing steps S1340 and S 1350 of FIG. 13 in detail.
FIG. 17 is an embodiment showing step S1330 of FIG. 13 in detail.
FIG. 18 is an embodiment showing steps S1340 and S 1350 of FIG. 13 in detail.

### DETAILED DESCRIPTION OF THE INVENTION

Since the present invention may make various modifications and have various embodiments, specific embodiments will be illustrated in the drawings and described in detail. However, this is not intended to limit the present invention to the specific embodiments, and it should be understood that this includes all modifications, equivalents, or substitutes included in the spirit and technical scope of the present invention.

The terms such that include ordinal numbers, such as second and first may be used to describe various components, but the components should not be limited by these terms. The terms are used only for the purpose of distinguishing one component from another component. For example, without departing from the scope of the present invention, a second component may be referred to as a first component, and likewise, the first component may also be referred to as the second component. The term "and/or" includes a combination of a plurality of related/described items or any one of the plurality of related/described items.

In case a certain component is referred to as being "linked" or "connected" to another component, it should be understood that there may be other component between them, although the certain component may be directly linked to or connected to another component. On the other hand, in case a certain component is referred to as being "directly linked" or "directly connected" to another component, it should be understood that there are no other component between them.

The terms used in the present application are used merely to describe specific embodiments and are not intended to limit the present invention. A singular expression covers a plural expression unless the context clearly indicates otherwise. In the present application, the terms such as "comprise" or "have" are intended to designate the presence of a feature, number, step, operation, component, part, or combination thereof described in the specification, but should be understood not to preclude the presence or additional possibility of one or more other features, numbers, steps, operations, components, parts, or combinations thereof.

Unless defined otherwise, all terms used herein, including technical or scientific terms, have the same meaning as generally understood by a person who has an ordinary knowledge in the technical field to which the present invention belongs. The terms such as those generally defined in the dictionary should be interpreted as having a meaning consistent with the meaning of the context of the relevant technology, and shall not be interpreted in an ideal or overly formal sense unless defined in the present application explicitly.

Hereinafter, with reference to the attached drawings, embodiments will be described in detail. Regardless of the drawing symbol, the same or corresponding components are assigned to the same reference number, and duplicate descriptions thereof will be omitted.

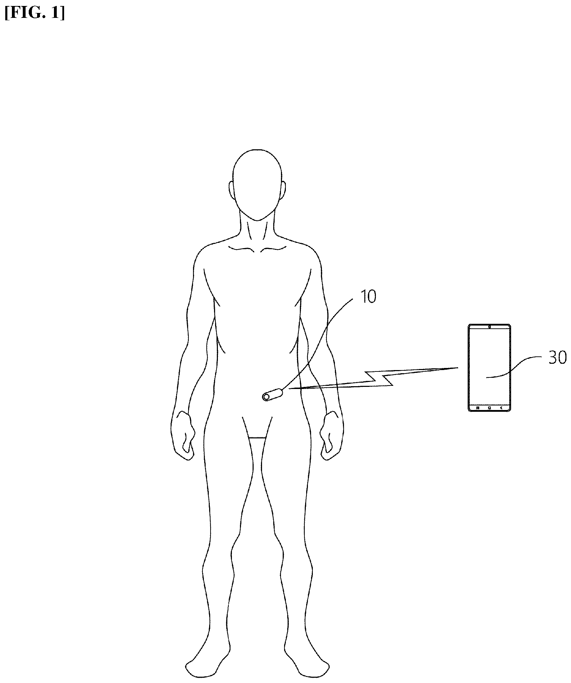

FIG. 1 is a schematic diagram illustrating a continuous glucose monitoring system according to an embodiment of the present invention.

Referring to FIG. 1, the continuous glucose monitoring system according to an embodiment of the present invention comprises a blood glucose measurement device 10 and a user terminal 30.

The blood glucose measurement device 10 is attached to the body of a user, i.e., a subject. A part of the blood glucose measurement device 10 may be inserted into the skin when attached to the body. The blood glucose measurement device 10 having the part inserted into the skin may measure blood glucose by periodically extracting body fluid. In an embodiment, the body fluid may be interstitial fluid. The blood glucose measurement device 10 may be expressed as a transmitter.

The user terminal 30 receives blood glucose information from the blood glucose measurement device 10. The user terminal 30 may mean a terminal device that can display the received blood glucose information to the user. The user terminal 30 may include a mobile terminal device that can communicate with the blood glucose measurement device 10, such as a smart phone, a tablet PC, or a notebook computer. However, the user terminal 30 is not limited thereto, and any terminal device that includes a communication function and is capable of installing a program or application may be included in the user terminal 30. The user terminal 30 may be used to mean a terminal used by a third party in addition to the terminal used by the subject. The user terminal 30 may be expressed as an external device.

The blood glucose measurement device 10 may transmit blood glucose information measured periodically at the request of the user terminal 30 or at preset every time to the user terminal 30. In this case, the blood glucose measurement device 10 may be connected to the user terminal 30 in a wired communication manner such as a USB cable for data communication, or may be connected to the user terminal 30 in a wireless communication manner such as infrared communication, NFC (Near Field Communication), Bluetooth, WiFi (Wireless Fidelity), LTE, etc.

The blood glucose measurement device 10 may convert a blood glucose measurement signal into blood glucose measurement data in order to transmit blood glucose information to the user terminal 30. The blood glucose measurement signal may be an analog signal measured by a sensor, and the blood glucose measurement data may be a digital signal.

Further, the blood glucose measurement device 10 may store the blood glucose measurement data in order to transmit blood glucose information to the user terminal 30. The blood glucose measurement device 10 may transmit the blood glucose measurement data to the user terminal 30 based on a request from the user terminal 30 or a predetermined transmission rule after storing the blood glucose measurement data.

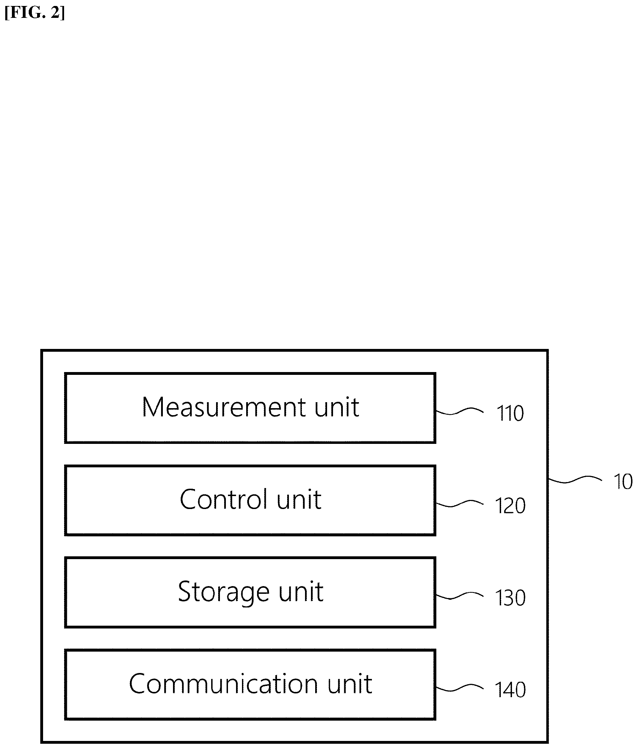

FIG. 2 is a diagram for explaining configurations of a blood glucose measurement device according to an embodiment of the present invention.

Referring to FIG. 2, the blood glucose measurement device 10 according to an embodiment of the present invention may comprise a measurement unit 110, a control unit 120, a storage unit 130, and a communication unit 140.

First, the measurement unit 110 measures a biometric signal of a subject. The biometric signal may include a blood glucose measurement signal. The measurement unit 110 may be inserted into a part of the body of the subject to measure a blood glucose concentration of the subject and generate the blood glucose measurement signal.

One end of the measurement unit 110 may be inserted into a skin of the subject to extract body fluid and measure blood glucose. A part of the measurement unit 110 inserted into a part of the subject's body may be a needle.

The measurement unit 110 may periodically extract body fluid and measure blood glucose. For example, the measurement unit 110 may extract body fluid at time intervals of 10 seconds and measure blood glucose. The measurement unit 110 may measure a plurality of blood glucose levels per one cycle of extracting body fluid. For example, the measurement unit 110 may measure blood glucose levels 30 times for 1 second per one cycle. The measurement unit 110 may periodically extract body fluid and measure a plurality of blood glucose levels per one cycle. For example, the measurement unit 110 may measure blood glucose 30 times between 14 minutes 00 seconds and 14 minutes 01 seconds, measure blood glucose 30 times between 14 minutes 11 seconds and 14 minutes 12 seconds, and measure blood glucose 30 times between 14 minutes 22 seconds and 14 minutes 23 seconds.

Next, the control unit 120 may convert the biometric signal, which is an analog signal, into biometric data, which is a digital signal. The control unit 120 may convert the blood glucose measurement signal into digital data to generate blood glucose measurement data.

Next, the control unit 120 may determine the validity of the blood glucose measurement data stored in a plurality of storage areas. The control unit 120 may use a predetermined rule to determine the validity of the blood glucose measurement data. According to an embodiment, the control unit 120 may determine the validity of the blood glucose measurement data using a parity bit. If the value of the parity bit has an error value, the control unit 120 may determine that a problem has occurred in the validity of the blood glucose measurement data. According to another embodiment, the control unit 120 may determine the validity of the blood glucose measurement data using a CRC (cyclic redundancy check) algorithm. According to another embodiment, the control unit 120 may determine the validity of the blood glucose measurement data using a predetermined data value inserted to or combined with the blood glucose measurement data. For example, the control unit 120 may store error detection data together with the blood glucose measurement data to have a predetermined value at a front end and/or a back end of the blood glucose measurement data when storing the blood glucose measurement data, and the control unit 120 may determine the validity of the blood glucose measurement data depending on whether the error detection data stored at the front end and/or the back end of the blood glucose measurement data has a preset value. In addition, various methods for determining the validity may be used by those skilled in the art. Meanwhile, information used for such validity determination may be stored together in connection with the corresponding data when storing the blood glucose measurement data.

Next, the control unit 120 may control the overall operation of the transmitter. The control unit 120 may control the operation of the measurement unit 110, the communication unit 140, and the storage unit 130.

The control unit 120 may control the blood glucose measurement operation of the measurement unit 110. The control unit 120 may control intervals at which the measurement unit 110 extracts blood glucose, the number of times blood glucose is extracted per a cycle, etc.

The control unit 120 may control the storage unit 130 to store the biometric data. The control unit 120 may control the storage unit 130 to store the blood glucose measurement data.

According to an embodiment, the control unit 120 may control to store the blood glucose measurement data in a first storage area included in a plurality of storage areas of the storage unit 130. Also, the control unit 120 may control to store the blood glucose measurement data in a second storage area included in the plurality of storage areas of the storage unit 130. In this case, the blood glucose measurement data stored in the first storage area may match with the blood glucose measurement data stored in the second storage area. In other words, the blood glucose measurement data stored in the first storage area and the blood glucose measurement data stored in the second storage area may be the same data.

According to an embodiment, the control unit 120 may control to store the blood glucose measurement data in the first storage area included in the plurality of storage areas of the storage unit 130. The control unit 120 may determine whether a preset error event has occurred in the first storage area, and if it is determined that the error event has occurred, the control unit 120 may control to store the blood glucose measurement data in the second storage area included in the plurality of storage areas of the storage unit 130.

According to an embodiment, the control unit 120 may store the blood glucose measurement data by crossing the first storage area and the second storage area included in the plurality of storage areas of the storage unit 130. Specifically, the control unit 120 may control to store first blood glucose measurement data in a first storage space of the first storage area. Also, the control unit 120 may control to store second blood glucose measurement data in a second storage space of the second storage area.

According to an embodiment,, the first storage space and the second storage space of the first storage area may correspond to the first storage space and the second storage space of the second storage area, respectively. The first storage space of the first storage area may be set to correspond to the first storage space of the second storage area. The second storage space of the first storage area may be set to correspond to the second storage space of the second storage area. For example, if a storage space having an index of 0 to an index of 63 is set in the first storage area, a storage space having an index of 0 to an index of 63 may also be set in the second storage area. And, if a first storage space having a storage space having an index of 0 to an index of 9 is set in the first storage area, a first storage space having a storage space having an index of 0 to an index of 9 may also be set in the second storage area to correspond to the first storage space of the first storage area.

In addition, the first storage space and the second storage space of the first storage area may be continuous with each other. The first storage space and the second storage space of the second storage area may be continuous with each other. For example, if a first storage space having storage spaces from index 0 to index 9 is set in the first storage area, the second storage space of the first storage area may be set to have storage spaces from index 10 to index 19. Accordingly, the first storage space and the second storage space of the first storage area may have consecutive storage spaces from index 0 to index 19.

According to an embodiment, the control unit 120 may control to store the first blood glucose measurement data in the first storage space of the first storage area of the storage unit 130. In addition, the control unit 120 may control to store second blood glucose measurement data in the first storage space of the second storage area of the storage unit 130. Herein, the first storage space of the first storage area may correspond to the first storage space of the second storage area.

The control unit 120 may control the operation of the communication unit 140 to transmit biometric data to an external device.

The control unit 120 may check the validity of the blood glucose measurement data stored in the first storage area; and if the blood glucose measurement data stored in the first storage area is determined to be invalid, transmit the blood glucose measurement data stored in the second storage area to the external device.

The control unit 120 may control the communication unit 140 to transmit the blood glucose measurement data stored in at least one of the plurality of storage areas to the external device, based on a result of determining the validity of the blood glucose measurement data.

The control unit 120 may check the validity of the blood glucose measurement data stored in the first storage area and the second storage area, and transmit the blood glucose measurement data determined to be valid among the blood glucose measurement data stored in the first storage area and the second storage area to the external device.

Next, the storage unit 130 may store biometric data. The storage unit 130 may store the blood glucose measurement data. The storage unit 130 may store the blood glucose measurement data depending on the control of the control unit 120.

The storage unit 130 may include a memory capable of storing data. According to an embodiment, the storage unit 130 may be a non-volatile memory, but is not limited thereto.

The storage unit 130 may include a plurality of storage areas. According to an embodiment, the plurality of storage areas may refer to different memory devices that are physically divided from each other. That is, the first storage area and the second storage area may be memory devices different from each other. According to another embodiment, the plurality of storage areas may refer to areas logically and/or physically divided in one memory device. For example, the first storage area and the second storage area may mean a sector and/or sets of the sector which are different from each other. Herein, the sector may include both a physical sector and a logical sector.

The storage area may include a plurality of storage spaces. For example, the storage space may mean a space capable of storing data of 1 byte. Assuming that the first storage area is a sector of 2 KB size, the first storage area may consist of 2048 storage spaces of 1 byte size.

Next, the communication unit 140 may transmit biometric data to an external device. The communication unit 140 may transmit the blood glucose measurement data to the external device. The communication unit 140 may transmit the blood glucose measurement data stored in at least one of a plurality of storage areas to the external device.

The communication unit 140 may include an infrared communication module, an NFC (Near Field Communication) module, a Bluetooth module, a WiFi (Wireless Fidelity) module, an LTE module, etc.

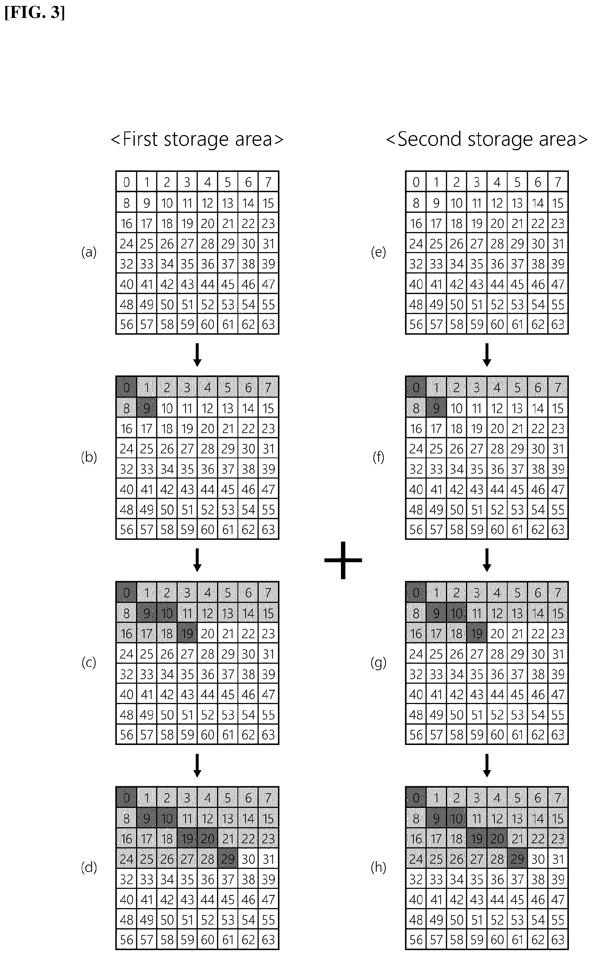

FIG. 3 is a diagram for explaining the first embodiment of a blood glucose data storage process according to an embodiment of the present invention.

FIG. 3(a) and FIG. 3(e) are diagrams illustrating a storage area of a memory used for data storage in rows and columns. FIG. 3(a) shows a storage area illustrated in eight rows and eight columns. Each cell in FIG. 3(a) represents a size of 1 byte, and therefore, the storage area of the memory illustrated in the diagram represents a storage area of the memory having a storage capacity having a size of 64 bytes. The expression "memory" in the description may be used interchangeably with the storage unit 130 described above. In addition, the expression "memory" in the description may be used interchangeably with a term indicating a storage space unit of the memory. For example, the matrix illustrating the storage area of the memory in FIG. 3(a) and FIG. 3(e) may mean a sector for the data storage. Herein, the sector may include both a physical sector and a logical sector. The matrices of FIG. 3(a) and FIG. 3(e) represent a state in which no data is currently stored. In addition, FIG. 3(a) and FIG. 3(e) representing a first storage area may mean physical and/or logical storage spaces different from each other within the same memory device. For example, a first sector within one memory device may be a first storage area, and a second sector may be a second storage area. In addition, FIG. 3(a) representing the first storage area and FIG. 3(e) representing the second storage area may mean physical and/or logical storage spaces within the memory devices different from each other. For example, a sector of a first memory device may be the first storage area, and a sector of a second memory device may be the second storage area.

The diagram illustrated in FIG. 3 shows a process of storing first blood glucose measurement data, second blood glucose measurement data, and third blood glucose measurement data. Herein, the first blood glucose measurement data, the second blood glucose measurement data, and the third blood glucose measurement data may refer to information measuring blood glucose of a subject at different points of time. For example, it is assumed that the blood glucose of the subject is measured 30 times per second and the measurement is repeated at intervals of 10 seconds. That is, there may be first glucose measurement 30 times between 0 and 1 second, second blood glucose measurement 30 times between 11 and 12 seconds, which is 10 seconds later, and third blood glucose measurement 30 times between 22 and 23 seconds, which is 10 seconds later. In this case, as an example, the first blood glucose measurement data may be data regarding blood glucose measurement of the first 30 times, the second blood glucose measurement data may be data regarding blood glucose measurement of the second 30 times, and the third blood glucose measurement data may be data regarding blood glucose measurement of the third 30 times. Also, as another example, the first blood glucose measurement data may be data regarding blood glucose measurement of one time among blood glucose measurement of the first 30 times, the second blood glucose measurement data may be data regarding blood glucose measurement of two times among blood glucose measurement of the first 30 times, and the third blood glucose measurement data may be data regarding blood glucose measurement of three times among blood glucose measurement of the first 30 times.

The control unit 120 may control the storage unit 130 to convert the blood glucose measurement signal measured by a sensor unit into digital data, i.e., the blood glucose measurement data, and then store the blood glucose measurement data in the storage area of the memory illustrated in FIGS. 3(a) and 3(e).

First, as shown in FIG. 3(b), the control unit 120 may control to store the first blood glucose measurement data in the storage spaces from the 0^{th} index to the 9^{th} index of the first storage area of the storage unit 130. Next, as shown in FIG. 3(c), the control unit 120 may control to store the second blood glucose measurement data in the storage spaces from the 10^{th} index to the 19^{th} index of the first storage area of the storage unit 130. Next, as shown in FIG. 3(d), the control unit 120 may control to store the third blood glucose measurement data in the storage spaces from the 20^{th} index to the 29^{th} index of the first storage area of the storage unit 130.

Also, as shown in FIG. 3(f), the control unit 120 may control to store the first blood glucose measurement data in the storage spaces from 0^{th} index 0 to the 9^{th} index of the second storage area of the storage unit 130. Next, as shown in FIG. 3(g), the control unit 120 may control to store the second blood glucose measurement data in the storage spaces from the 10^{th} index to the 19^{th} index of the second storage area of the storage unit 130. Next, as shown in FIG. 3(h), the control unit 120 may control to store the third blood glucose measurement data in the storage spaces from the 20^{th} index to the 29^{th} index of the second storage area of the storage unit 130.

As such, both the first storage area and the second storage area store the first blood glucose measurement data, the second blood glucose measurement data, and the third blood glucose measurement data in the same manner. Through this storage manner, by storing the same blood glucose measurement data in at least two storage areas, respectively, the blood glucose measurement device 10 has at least one backup data. Therefore, even if a data error occurs in one storage area, data stored in another storage area can be used as the backup data, thereby minimizing data loss due to the occurrence of an error and enhancing data reliability.

Meanwhile, only the first storage area and the second storage area are illustrated and described herein, but the blood glucose measurement data may be stored in two or more storage areas through the above process.

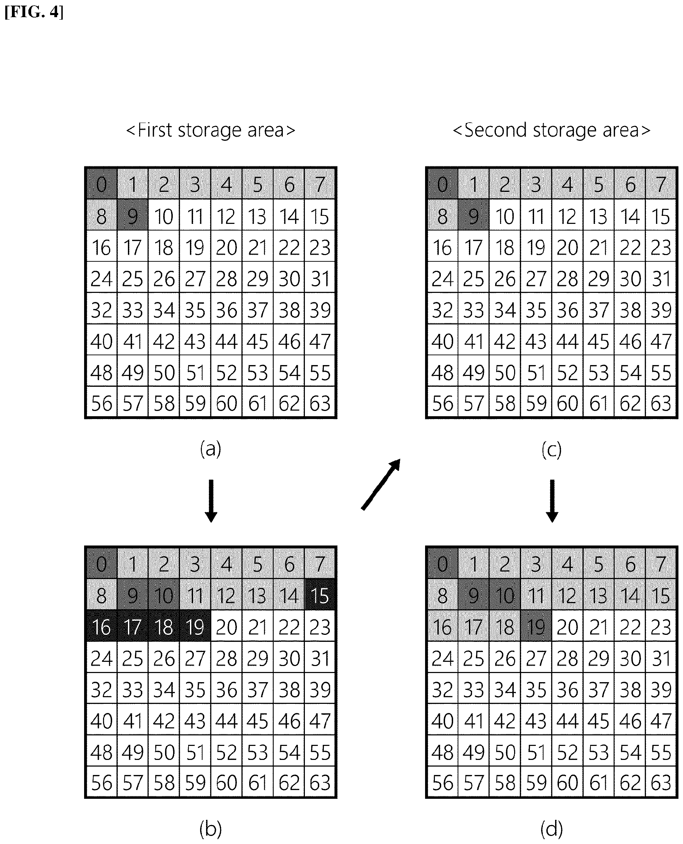

FIG. 4 is a diagram for explaining the second embodiment of a blood glucose data storage process according to an embodiment of the present invention.

FIG. 4 describes a process for storing blood glucose measurement data. Below, an embodiment will be described with reference to FIG. 4, and the explanation of parts that overlap with the previously described contents will be omitted.

First, as shown in FIG. 4(a), the control unit 120 may control the storage unit 130 to store the first blood glucose measurement data in the storage spaces from the 0^{th} index to the 9^{th} index included in the first storage area of the storage unit 130.

Next, as shown in FIG. 4(b), the control unit 120 may control to store the second blood glucose measurement data in the storage spaces from the 10^{th} index to the 19^{th} index included in the first storage area of the storage unit 130. In this case, if a specific event occurs and data storage is stopped after the second blood glucose measurement data is stored in the storage spaces from the 10^{th} index to the 14^{th} index, the control unit 120 may not store any more data related to the second blood glucose measurement data in the storage spaces from the 15^{th} index to the 19^{th} index. In this case, the control unit 120 may store dummy data in the storage spaces from the 15^{th} index to the 19^{th} index. Herein, a specific event may include a case where the power supply is temporarily interrupted due to an external electromagnetic wave, a case where the power supply of a battery is temporarily interrupted due to an external impact, etc. In addition, the specific event may include various events that contribute to the interruption of the data storage process. For example, there is an example where the number of deletion/recordation times is exceeded or a problem occurs in the validity of the storage area.

Then, as shown in FIG. 4(c), the control unit 120 may control to store the third blood glucose measurement data in the storage spaces from the 0^{th} index to the 9^{th} index included in the second storage area of the storage unit 130. In other words, even if there is a storage space that can store data in the first storage area, the control unit 120 may control to store subsequent blood glucose measurement data in the second storage area without storing additional blood glucose measurement data anymore.

Next, as shown in FIG. 4(d), the control unit 120 may control to store the fourth blood glucose measurement data in the storage spaces from the 10^{th} index to the 19^{th} index of the second storage area of the storage unit 130.

In this case, even if an error occurs in one of the storage areas, data stored in other storage areas can be used so that data loss due to the occurrence of an error can be minimized and data reliability can be enhance through this.

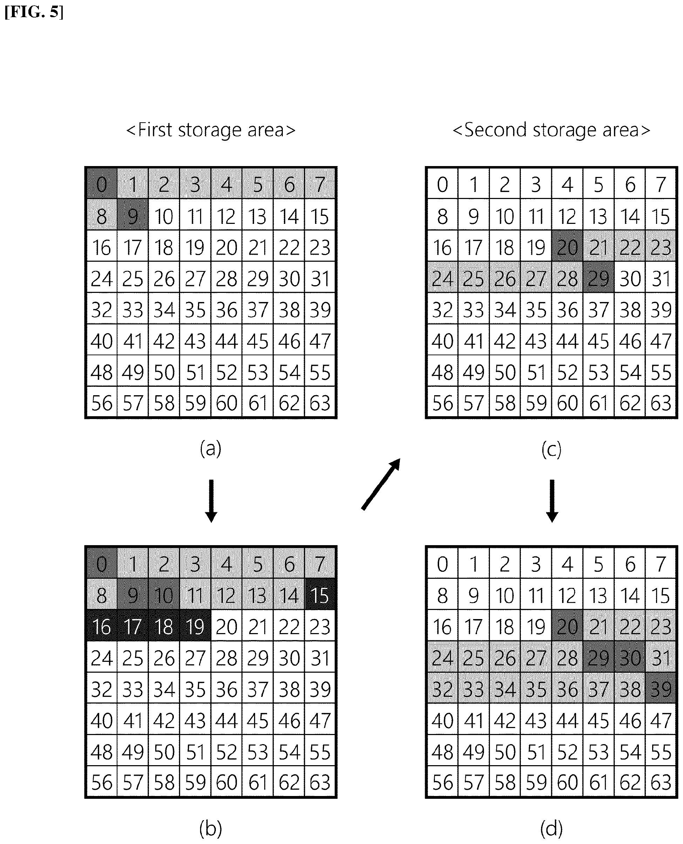

FIG. 5 is a diagram for explaining the third embodiment of a blood glucose data storage process according to an embodiment of the present invention.

FIG. 5 describes a process for storing blood glucose measurement data. Below, an embodiment will be described with reference to FIG. 5, but the explanation of parts that overlap with the previously explained contents will be omitted.

First, as shown in FIG. 5(a), the control unit 120 may control the storage unit 130 to store the first blood glucose measurement data in the storage spaces from the 0^{th} index to the 9^{th} index included in the first storage area of the storage unit 130.

Next, as shown in FIG. 5(b), the control unit 120 may control to store the second blood glucose measurement data in the storage spaces from the 10^{th} index to the 19^{th} index included in the first storage area of the storage unit 130. In this case, if a specific event occurs and data storage is stopped after the second blood glucose measurement data is stored in the storage spaces from the 10^{th} index to the 14^{th} index, the control unit 120 may no longer store data related to the second blood glucose measurement data in the storage spaces from the 15^{th} index to the 19^{th} index. In this case, the control unit 120 may store dummy data in the storage spaces from the 15^{th} index to the 19^{th} index. Herein, the specific event may include a case where the power supply is temporarily interrupted due to an external electromagnetic wave, a case where the power supply of a battery is temporarily interrupted due to an external shock, etc. In addition, the specific event may include various events that contribute to the interruption of the data storage process. For example, there are examples where the number of deletion/recordation times is exceeded or a problem occurs in the validity of the corresponding storage area.

Then, as shown in FIG. 5(c), the control unit 120 may control to store the third blood glucose measurement data in the storage spaces from the 20^{th} index to the 29^{th} index included in the second storage area of the storage unit 130. In other words, the control unit 120 may control to store subsequent blood glucose measurement data in the second storage area without storing additional blood glucose measurement data anymore, even if there is a storage space capable of storing data in the first storage area.

Next, as shown in FIG. 5(d), the control unit 120 may control to store the fourth blood glucose measurement data in the storage spaces from the 30^{th} index to the 39^{th} index of the second storage area of the storage unit 130.

In this case, even if an error occurs in one of the storage areas, data stored in other storage areas can be used so that data loss due to the occurrence of an error can be minimized and data reliability can be enhanced.

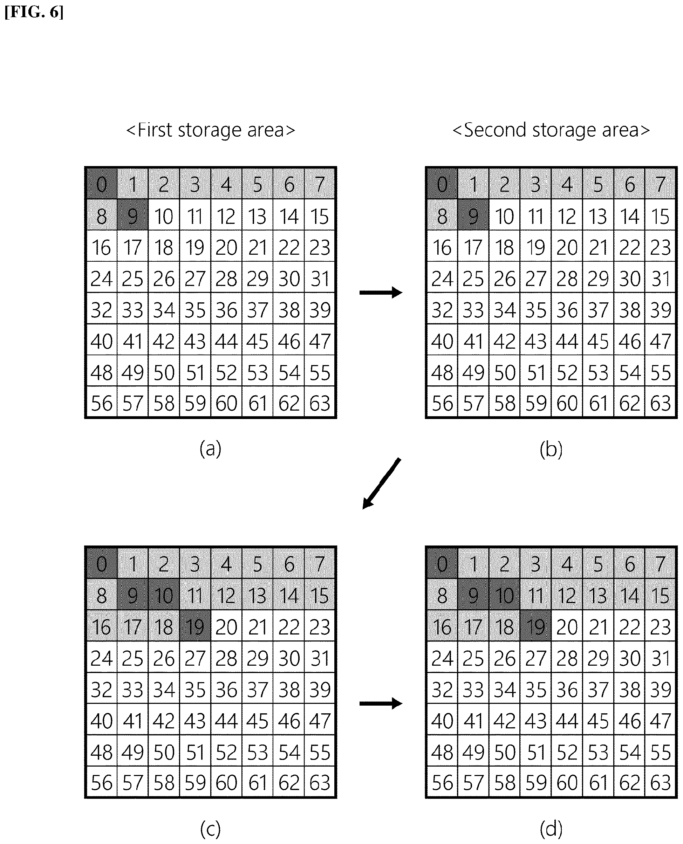

FIG. 6 is a diagram for explaining the fourth embodiment of a blood glucose data storage process according to an embodiment of the present invention.

FIG. 6 describes a process for storing blood glucose measurement data. Below, an embodiment will be described with reference to FIG. 6, but the explanation of parts that overlap with the previously described contents will be omitted.

First, as shown in FIG. 6(a), the control unit 120 may control to store the first blood glucose measurement data in the storage spaces from the 0^{th} index to the 9^{th} index included in the first storage area of the storage unit 130.

Next, as shown in FIG. 6(b), the control unit 120 may control to store the second blood glucose measurement data in the storage spaces from the 0^{th} index to the 9^{th} index included in the second storage area of the storage unit 130.

Next, as shown in FIG. 6(c), the control unit 120 may control to store the third blood glucose measurement data in the storage spaces from the 10^{th} index to the 19^{th} index included in the first storage area of the storage unit 130.

Next, as shown in FIG. 6(d), the control unit 120 may control to store the fourth blood glucose measurement data in the storage spaces from the 10^{th} index to the 19^{th} index included in the second storage area of the storage unit 130.

That is, the control unit 120 may control to store a plurality of blood glucose measurement data by crossing the first storage area and the second storage area sequentially. As an example, in case of three or more storage areas, the control unit 120 may control to store the plurality of blood glucose measurement data by crossing the three or more storage areas sequentially.

In this case, even if an error occurs in one of the storage areas, data stored in other storage areas can be used so that data loss due to the occurrence of an error can be minimized and data reliability can be enhanced.

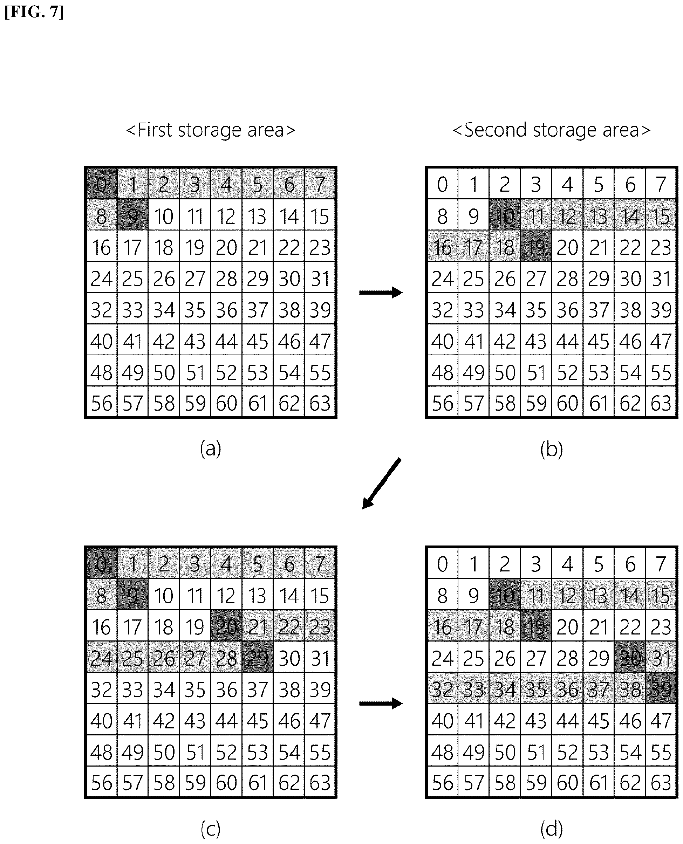

FIG. 7 is a diagram for explaining the first embodiment of a blood glucose data storage process according to an embodiment of the present invention.

FIG. 7 describes a process for storing blood glucose measurement data. Below, an embodiment will be described with reference to FIG. 7, but the explanation of parts that overlap with the previously described contents will be omitted.

First, as shown in FIG. 7(a), the control unit 120 may control to store the first blood glucose measurement data in the storage spaces from the 0^{th} index to the 9^{th} index included in the first storage area of the storage unit 130.

Next, as shown in FIG. 7(b), the control unit 120 may control to store the second blood glucose measurement data in the storage spaces from the 10^{th} index to the 19^{th} index included in the second storage area of the storage unit 130. That is, the control unit 120 may control not to store data in the storage space of the second storage area (the storage spaces from the 0^{th} index to the 9^{th} index) corresponding to the storage spaces of the first storage area (the storage spaces from the 0^{th} index to the 9^{th} index) in which the first blood glucose measurement data is stored, and to store the second blood glucose measurement data in the storage spaces of the second storage area (the storage spaces from the 10^{th} index to the 19^{th} index) that is subsequent thereafter.

Next, as shown in FIG. 7(c), the control unit 120 may control to store the third blood glucose measurement data in the storage spaces from the 20^{th} index to the 29^{th} index included in the first storage area of the storage unit 130. That is, The control unit 120 may control not to store data in the storage spaces of the first storage area (the storage spaces from the 10^{th} index to the 19^{th} index) corresponding to the storage spaces of the second storage area (the storage spaces from the 10^{th} index to the 19^{th} index) in which the second blood glucose measurement data is stored, and to store the third blood glucose measurement data in the storage spaces of the first storage area (the storage spaces from the 20^{th} index to the 29^{th} index) that is subsequent thereafter.

Next, as shown in FIG. 7(d), the control unit 120 may control to store the fourth blood glucose measurement data in the storage spaces from the 30^{th} index to the 39^{th} index included in the second storage area of the storage unit 130. That is, the control unit 120 may control not to store data in the storage spaces of the second storage area (the storage spaces from the 20^{th} index to the 29^{th} index) corresponding to the storage spaces of the first storage area (the storage spaces from the 20^{th} index to the 29^{th} index) in which the third blood glucose measurement data is stored, and to store the fourth blood glucose measurement data in the storage spaces of the second storage area (the storage spaces from the 30^{th} index to the 39^{th} index) that is subsequent thereafter.

That is, the control unit 120 may control to store a plurality of blood glucose measurement data by sequentially crossing the first storage area and the second storage area. As an example, in case of three or more storage areas, the control unit 120 may also control to store the plurality of blood glucose measurement data by sequentially crossing the three or more storage areas.

In this case, even if an error occurs in any one of the storage areas, data stored in other storage areas can be used so that data loss due to the occurrence of an error can be minimized and data reliability can be enhanced through this.

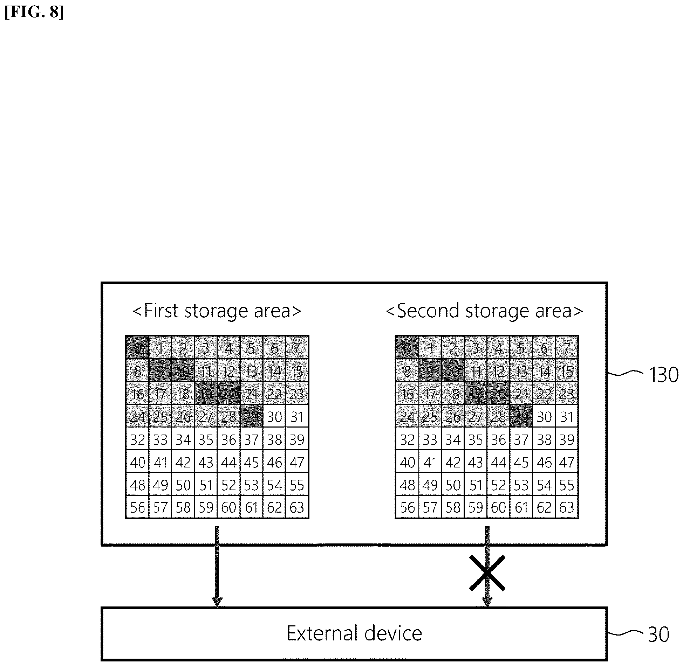

FIG. 8 is the first embodiment for explaining a blood glucose measurement data transmission process according to an embodiment of the present invention.

FIG. 8 is related to the embodiment described in FIG. 3. In FIG. 8, it is assumed that the storage unit 130 stores information on the first blood glucose measurement data in the storage spaces from the 0^{th} index to the 9^{th} index of the first storage area, stores information on the second blood glucose measurement data in the storage spaces from the 10^{th} index to the 19^{th} index, and stores information on the third blood glucose measurement data in the storage spaces from the 20^{th} index to the 29^{th} index. In addition, it is assumed that the storage unit 130 stores information on the first blood glucose measurement data in the storage spaces from the 0^{th} index to the 9^{th} index of the second storage area, stores information on the second blood glucose measurement data in the storage spaces from the 10^{th} index to the 19^{th} index, and stores information on the third blood glucose measurement data in the storage spaces from the 20^{th} index to the 29^{th} index. Herein, it is assumed that the blood glucose measurement data stored in the first storage area and the second storage area have no validity problem.

In order to transmit the blood glucose measurement data to an external device, first, the control unit 120 determines the validity of the first blood glucose measurement data, the second blood glucose measurement data, and the third blood glucose measurement data which are stored in the storage spaces from the 0^{th} index to the 29^{th} index of the first storage area. As a result of the validity determination, the control unit 120 determines that no validity problem has occurred in the first blood glucose measurement data, the second blood glucose measurement data, and the third blood glucose measurement data which are stored in the first storage area.

Then, the control unit 120 transmits the first blood glucose measurement data, the second blood glucose measurement data, and the third blood glucose measurement data which are stored in the storage spaces from the 0^{th} index to the 29^{th} index of the first storage area to an external device through the communication unit 140. In this case, the first blood glucose measurement data, the second blood glucose measurement data, and the third blood glucose measurement data which are stored in the storage spaces from the 0^{th} index to the 29^{th} index of the second storage area are not transmitted to the external device.

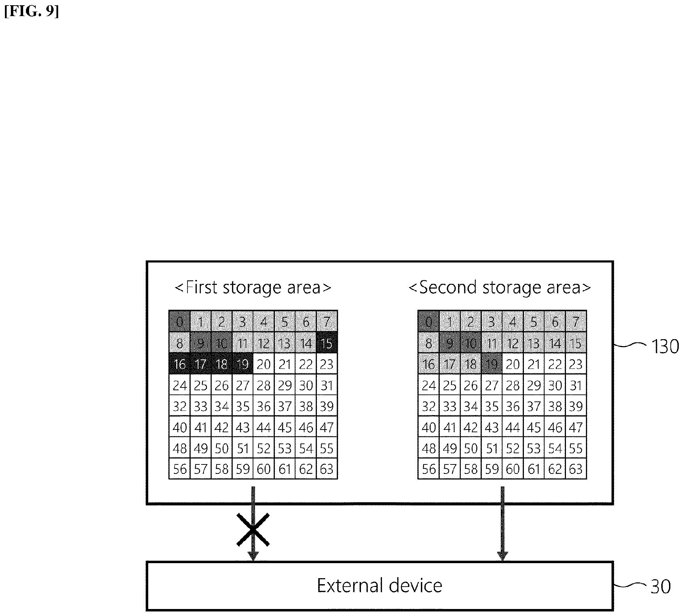

FIG. 9 is the second embodiment for explaining a blood glucose measurement data transmission process according to an embodiment of the present invention.

FIG. 9 is related to the embodiment described in FIG. 4. In FIG. 9, it is assumed that the storage unit 130 stores information on the first blood glucose measurement data in the storage spaces from the 0^{th} index to the 9^{th} index of the first storage area, and stores information on the second blood glucose measurement data in the storage spaces from the 10^{th} index to the 19^{th} index. Herein, it is assumed that the second blood glucose measurement data was not able to store blood glucose information from the storage spaces of the 15^{th} index due to an unstable power supply during storage, and thus dummy data was stored in the storage spaces from the 15^{th} index to the 19^{th} index. In addition, it is assumed that the storage unit 130 stores information on the third blood glucose measurement data in the storage spaces from the 0^{th} index to the 9^{th} index of the second storage area, and stores information on the fourth blood glucose measurement data in the storage spaces from the 10^{th} index to the 19^{th} index. Here, it is assumed that the blood glucose measurement data stored in the second storage area has no validity problem.

In order to transmit the blood glucose measurement data to an external device, first, the control unit 120 determines the validity of the first blood glucose measurement data and the second blood glucose measurement data stored in the storage spaces from the 0^{th} index to the 19^{th} index of the first storage area. As a result of the validity determination, the control unit 120 determines that a validity problem has occurred in the second blood glucose measurement data stored in the first storage area.

Also, the control unit 120 determines the validity of the third blood glucose measurement data and the fourth blood glucose measurement data stored in the storage spaces from the 0^{th} index to the 19^{th} index of the second storage area. The control unit 120 determines that there is no validity problem in the third blood glucose measurement data and the fourth blood glucose measurement data stored in the second storage area.

Then, the control unit 120 transmits the third blood glucose measurement data and the fourth blood glucose measurement data stored in the second storage area to the external device through the communication unit 140. That is, the first blood glucose measurement data and the second blood glucose measurement data stored in the first storage area are not transmitted to the external device.

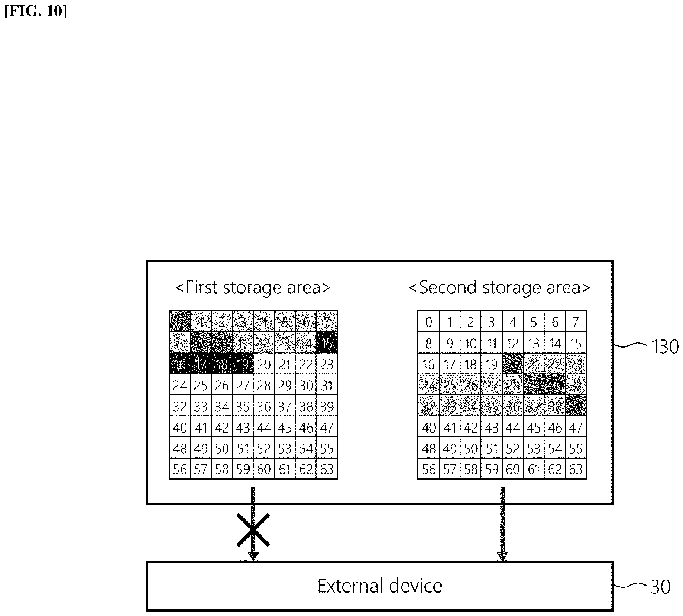

FIG. 10 is the third embodiment for explaining a blood glucose measurement data transmission process according to an embodiment of the present invention.

FIG. 10 is related to the embodiment described in FIG. 5. In FIG. 10, it is assumed that the storage unit 130 stores information on the first blood glucose measurement data in the storage spaces from the 0^{th} index to the 9^{th} index of the first storage area, and stores information on the second blood glucose measurement data in the storage spaces from the 10^{th} index to the 19^{th} index. Herein, it is assumed that the second blood glucose measurement data was not able to store blood glucose information from the storage space of the 15^{th} index due to an unstable power supply during storage, and thus dummy data was stored in the storage spaces from the 15^{th} index to the 19^{th} index. In addition, it is assumed that the storage unit 130 stores information on the third blood glucose measurement data in the storage spaces from the 20^{th} index to the 29^{th} index of the second storage area, and stores information on the fourth blood glucose measurement data in the storage spaces from the 30^{th} index to the 39^{th} index. Herein, it is assumed that the blood glucose measurement data stored in the second storage area has no validity problem.

In order to transmit the blood glucose measurement data to an external device, first, the control unit 120 determines the validity of the first blood glucose measurement data and the second blood glucose measurement data stored in the storage spaces from the 0^{th} index to the 19^{th} index of the first storage area. As a result of the validity determination, the control unit 120 determines that there is a validity problem in the second blood glucose measurement data stored in the first storage area.

Also, the control unit 120 determines the validity of the third blood glucose measurement data and the fourth blood glucose measurement data stored in the storage spaces from the 20^{th} index to the 39^{th} index of the second storage area. The control unit 120 determines that there is no validity problem in the third blood glucose measurement data and the fourth blood glucose measurement data stored in the second storage area.

Then, the control unit 120 transmits the third blood glucose measurement data and the fourth blood glucose measurement data stored in the second storage area to the external device through the communication unit 140. In other words, the first blood glucose measurement data and the second blood glucose measurement data stored in the first storage area are not transmitted to the external device.

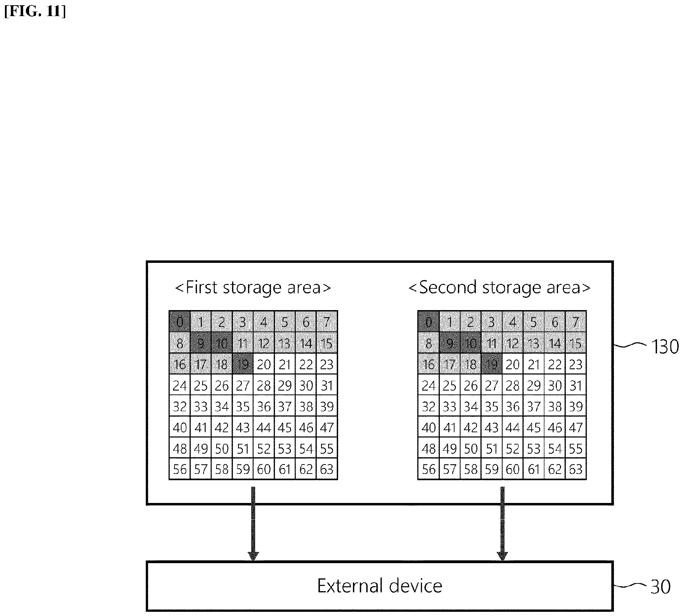

FIG. 11 is the fourth embodiment for explaining a blood glucose measurement data transmission process according to an embodiment of the present invention.

FIG. 11 is related to the embodiment described in FIG. 6. In FIG. 11, it is assumed that the storage unit 130 stores information on the first blood glucose measurement data in the storage spaces from the 0^{th} index to the 9^{th} index of the first storage area, and stores information on the third blood glucose measurement data in the storage spaces from the 10^{th} index to the 19^{th} index. In addition, it is assumed that the storage unit 130 stores information on the second blood glucose measurement data in the storage spaces from the 0^{th} index to the 9^{th} index of the second storage area, and stores information on the fourth blood glucose measurement data in the storage spaces from the 10^{th} index to the 19^{th} index. Herein, it is assumed that the blood glucose measurement data stored in the first storage area and the second storage area have no validity problem.

In order to transmit the blood glucose measurement data to an external device, first, the control unit 120 determines the validity of the first blood glucose measurement data and the third blood glucose measurement data stored in the storage spaces from the 0^{th} index to the 19^{th} index of the first storage area. As a result of the validity determination, the control unit 120 determines that there is no validity problem in the first blood glucose measurement data and the third blood glucose measurement data stored in the first storage area.

Also, the control unit 120 determines the validity of the second blood glucose measurement data and the fourth blood glucose measurement data stored in the storage spaces from the 0^{th} index to the 19^{th} index of the second storage area. As a result of the validity determination, the control unit 120 determines that there is no validity problem in the second blood glucose measurement data and the fourth blood glucose measurement data stored in the second storage area.

Then, the control unit 120 transmits the first blood glucose measurement data and the third blood glucose measurement data stored in the first storage area to the external device through the communication unit 140. In addition, the control unit 120 transmits the second blood glucose measurement data and the fourth blood glucose measurement data stored in the second storage area to the external device through the communication unit 140. If there is a validity problem in the first blood glucose measurement data and/or the third blood glucose measurement data stored in the first storage area, only the second blood glucose measurement data and the fourth blood glucose measurement data stored in the second storage area are transmitted to the external device.

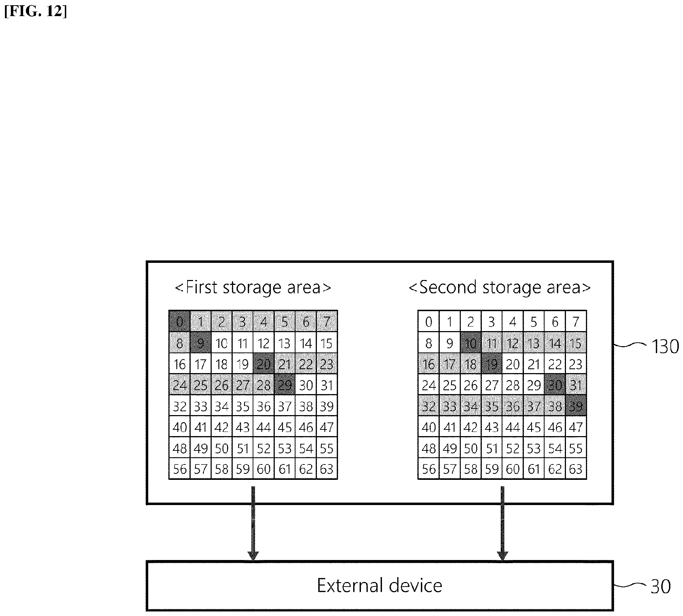

FIG. 12 is the fifth embodiment for explaining a blood glucose measurement data transmission process according to an embodiment of the present invention.

FIG. 12 is related to the embodiment described in FIG. 7. In FIG. 12, it is assumed that the storage unit 130 stores information on the first blood glucose measurement data in the storage spaces from the 0^{th} index to the 9^{th} index of the first storage area, and stores information on the third blood glucose measurement data in the storage spaces from the 20^{th} index to the 29^{th} index. In addition, it is assumed that the storage unit 130 stores information on the second blood glucose measurement data in the storage spaces from the 10^{th} index to the 19^{th} index of the second storage area, and stores information on the fourth blood glucose measurement data in the storage spaces from the 30^{th} index to the 39^{th} index. Herein, it is assumed that the blood glucose measurement data stored in the first storage area and the second storage area have no validity problems.

In order to transmit the blood glucose measurement data to an external device, first, the control unit 120 determines the validity of the first blood glucose measurement data and the third blood glucose measurement data stored in the storage spaces from the 0^{th} index to the 19^{th} index and the storage spaces from the 20^{th} index to the 29^{th} index of the first storage area. As a result of the validity judgment, the control unit 120 determines that there is no validity problem in the first blood glucose measurement data and the third blood glucose measurement data stored in the first storage area.

Also, in case the control unit 120 intends to transmit the blood glucose measurement data to an external device, the control unit 120 determines the validity of the second blood glucose measurement data and the fourth blood glucose measurement data stored in the storage spaces from the 10^{th} index to the 19^{th} index and the storage spaces from the 30^{th} index to the 39^{th} index of the second storage area. As a result of the validity determination, the control unit 120 determines that there is no validity problem in the second blood glucose measurement data and the fourth blood glucose measurement data stored in the second storage area.

Then, the control unit 120 transmits the first blood glucose measurement data and the third blood glucose measurement data stored in the first storage area to the external device through the communication unit 140. In addition, the control unit 120 transmits the second blood glucose measurement data and the fourth blood glucose measurement data stored in the second storage area to the external device through the communication unit 140. If there is a validity problem in the second blood glucose measurement data and/or the fourth blood glucose measurement data stored in the second storage area, only the first blood glucose measurement data and the third blood glucose measurement data stored in the first storage area are transmitted to the external device.

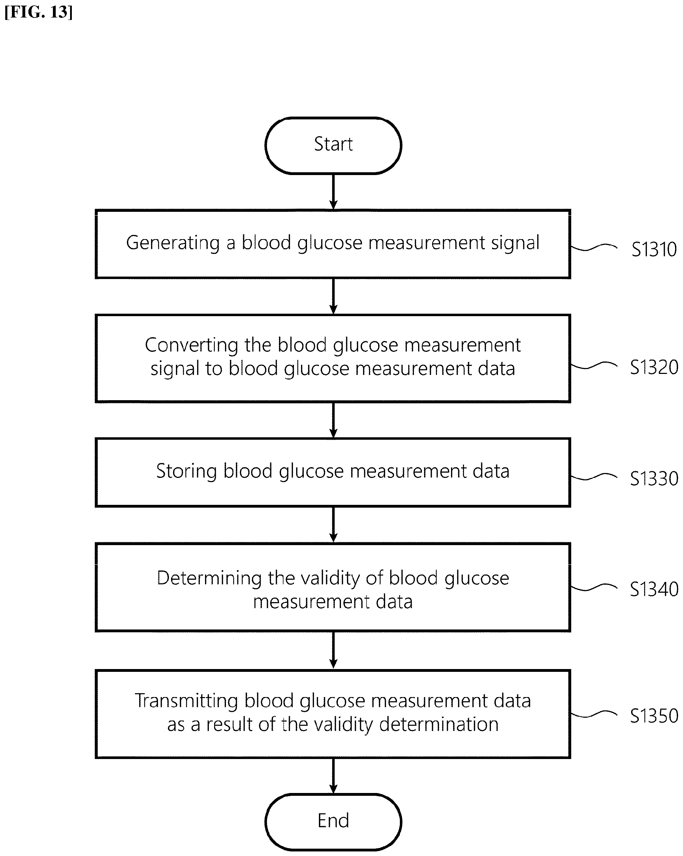

FIG. 13 is a flowchart illustrating a method for managing blood glucose measurement data according to an embodiment of the present invention.

The method for managing blood glucose measurement data according to FIG. 13 may be performed using the blood glucose measurement device described above.

Referring to FIG. 13, first, the measurement unit 110 measures blood glucose of a subject and generates a blood glucose measurement signal (S 1310).

Next, the control unit 120 converts the blood glucose measurement signal into blood glucose measurement data, which is a digital signal (S1320).

Next, the storage unit 130 stores the blood glucose measurement data in a plurality of storage areas (S1330). That is, the control unit 120 controls the storage unit 130 to store the blood glucose measurement data in the plurality of storage areas.

Next, the control unit 120 determines the validity of the blood glucose measurement data stored in the plurality of storage areas (S1340).

Next, the communication unit 140 transmits the blood glucose measurement data stored in at least one of the plurality of storage areas to an external device, based on a result of the determination (S1350). That is, the control unit 120 controls the communication unit 140 to transmit the blood glucose measurement data stored in at least one of the plurality of storage areas to the external device based on a result of the determination.

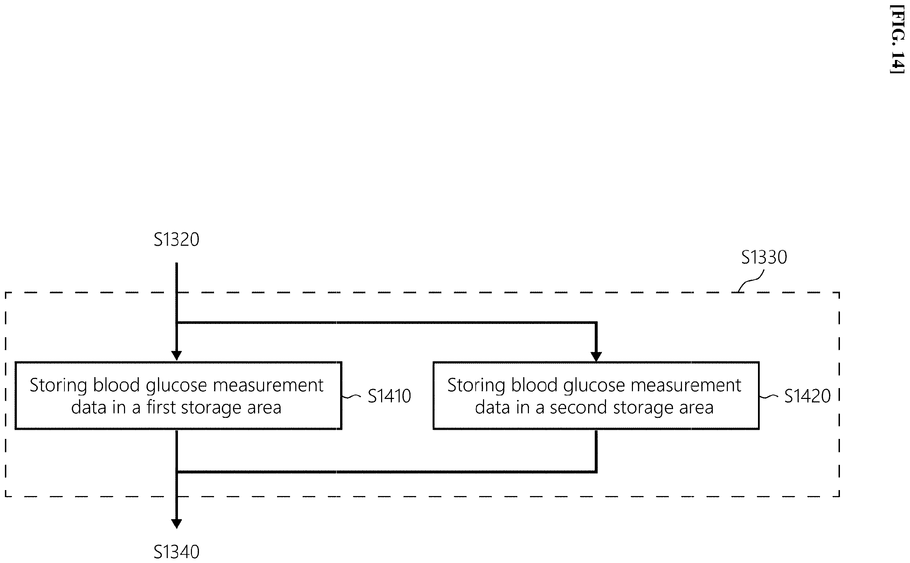

FIG. 14 is an embodiment showing step S1330 of FIG. 13 in detail.

Referring to FIG. 14, the storage unit 130 may store the blood glucose measurement data in a first storage area included in a plurality of storage areas (S1410). That is, the control unit 120 may control the storage unit 130 to store the blood glucose measurement data in the first storage area included in the plurality of storage areas.

Next, the storage unit 130 may store the blood glucose measurement data in a second storage area included in the plurality of storage areas (S1420). That is, the control unit 120 may control the storage unit 130 to store the blood glucose measurement data in the second storage area included in the plurality of storage areas.

In this case, the blood glucose measurement data stored in the first storage area may match with the blood glucose measurement data stored in the second storage area. That is, the storage unit 130 may store the same blood glucose measurement data as each other in the first storage area and the second storage area.

The steps S1410 and S1420 above may be performed sequentially and/or independently.

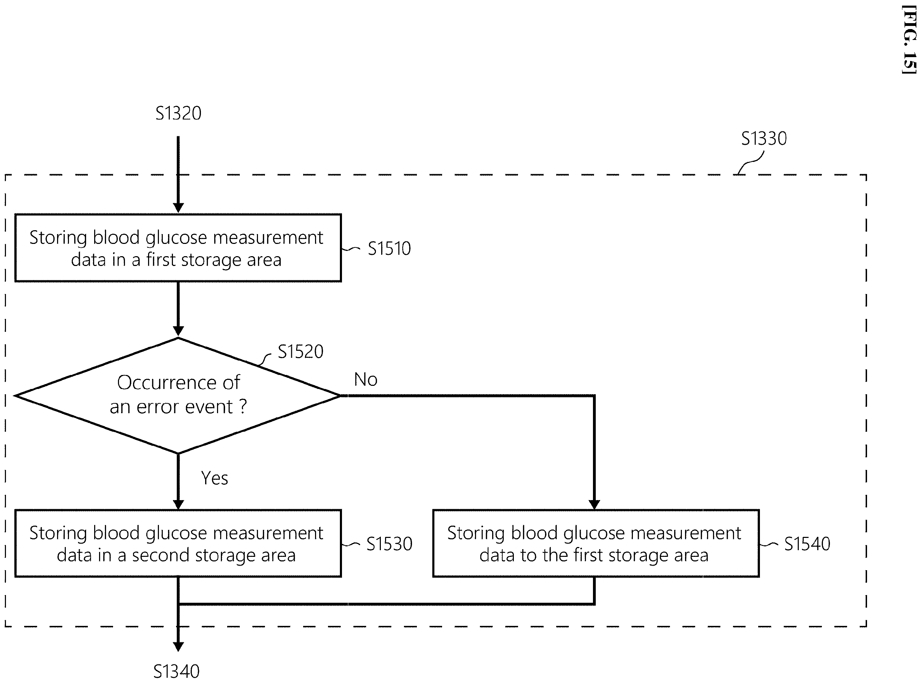

FIG. 15 is an embodiment showing step S1330 of FIG. 13 in detail.

Referring to FIG. 15, the storage unit 130 may store the blood glucose measurement data in the first storage area included in the plurality of storage areas. That is, the control unit 120 may control to store the blood glucose measurement data in the first storage area included in the plurality of storage areas (S1510).

Next, the control unit 120 may determine whether a preset error event has occurred in the first storage area (S1520).

If it is determined that the error event has occurred, the storage unit 130 may store the blood glucose measurement data in the second storage area included in the plurality of storage areas (S1530). That is, the control unit 120 may control the storage unit 130 to store the blood glucose measurement data in the second storage area included in the plurality of storage areas.

On the other hand, if it is determined that the error event has not occurred, the storage unit 130 may continue to store the blood glucose measurement data in the first storage area included in the plurality of storage areas (S1540). That is, the control unit 120 may control the storage unit 130 to continuously store the blood glucose measurement data in the first storage area included in the plurality of storage areas.

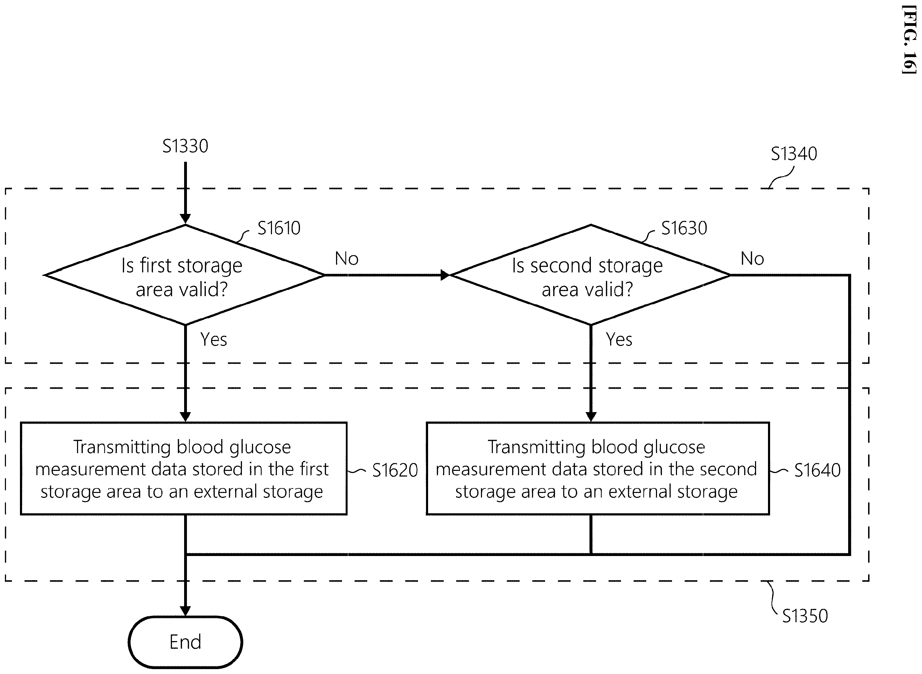

FIG. 16 is an embodiment showing steps S1340 and S 1350 of FIG. 13 in detail.

Referring to FIG. 16, in order to use the blood glucose measurement data, for example, to transmit the blood glucose measurement data to an external device, the control unit 120 may determine the validity of the blood glucose measurement data stored in the first storage area (S1610).

If the blood glucose measurement data stored in the first storage area is determined to be valid, the communication unit 140 may transmit the blood glucose measurement data stored in the first storage area to the external device (S1620). That is, the control unit 120 may control the communication unit 140 to transmit the blood glucose measurement data stored in the first storage area to the external device. In this case, the blood glucose measurement data stored in the second storage area is not used for transmission.

On the other hand, if the blood glucose measurement data stored in the first storage area is determined to be invalid, the control unit 120 may determine the validity of the blood glucose measurement data stored in the second storage area (S1630).

If the blood glucose measurement data stored in the second storage area is determined to be valid, the communication unit 140 may transmit the blood glucose measurement data stored in the second storage area to the external device (S1640). That is, the control unit 120 may control the communication unit 140 to transmit the blood glucose measurement data stored in the second storage area to the external device.

On the other hand, if the blood glucose measurement data stored in the second storage area is also determined to be invalid, the communication unit 140 may not transmit all of the blood glucose measurement data stored in the first storage area and the second storage area.

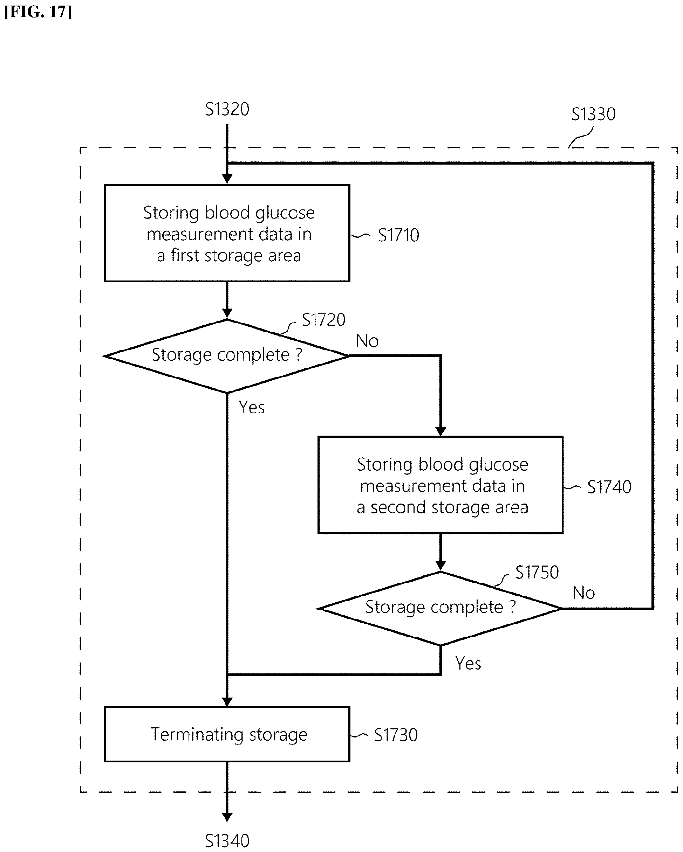

FIG. 17 is an embodiment showing step S1330 of FIG. 13 in detail.

Referring to FIG. 17, the storage unit 130 may store the blood glucose measurement data by crossing the first storage area and the second storage area included in a plurality of storage areas. That is, the control unit 120 may control the storage unit 130 to store the blood glucose measurement data by crossing the first storage area and the second storage area included in the plurality of storage areas.

First, the storage unit 130 may store the first blood glucose measurement data in the first storage area (S1710). That is, the control unit 120 may control the storage unit 130 to store the first blood glucose measurement data in the first storage area. According to an embodiment, the storage unit 130 may store the first blood glucose measurement data in a first storage space of the first storage area.

Next, the control unit 120 may determine whether there is data to be additionally stored in addition to the first blood glucose measurement data (S1720).

Then, if there is no more blood glucose measurement data to be stored, the control unit 120 may terminate the storage of the blood glucose measurement data (S1730).

On the other hand, if there is second blood glucose measurement data to be additionally stored, the storage unit 130 may store the second blood glucose measurement data in the second storage area (S1740). That is, the control unit 120 may control the storage unit 130 to store the second blood glucose measurement data in the second storage area. According to an embodiment, the storage unit 130 may store the second blood glucose measurement data in a second storage space of the second storage area. According to another embodiment, the storage unit 130 may store the second blood glucose measurement data in a first storage space of the second storage area.

Next, the control unit 120 may determine whether there is data to be additionally stored in addition to the second blood glucose measurement data (S 1750).

Then, if there is no more blood glucose measurement data to be stored, the control unit 120 may terminate the storage of the blood glucose measurement data (S1730).

On the other hand, if there is third blood glucose measurement data to be additionally stored, the storage unit 130 may store the third blood glucose measurement data in the first storage area. That is, the control unit 120 may control the storage unit 130 to store the third blood glucose measurement data in the first storage area.

Here, the first storage space and the second storage space of the first storage area may correspond to the first storage space and the second storage space of the second storage area, respectively. The first storage space and the second storage space may be continuous with each other. The first storage space of the first storage area may correspond to the first storage space of the second storage area.

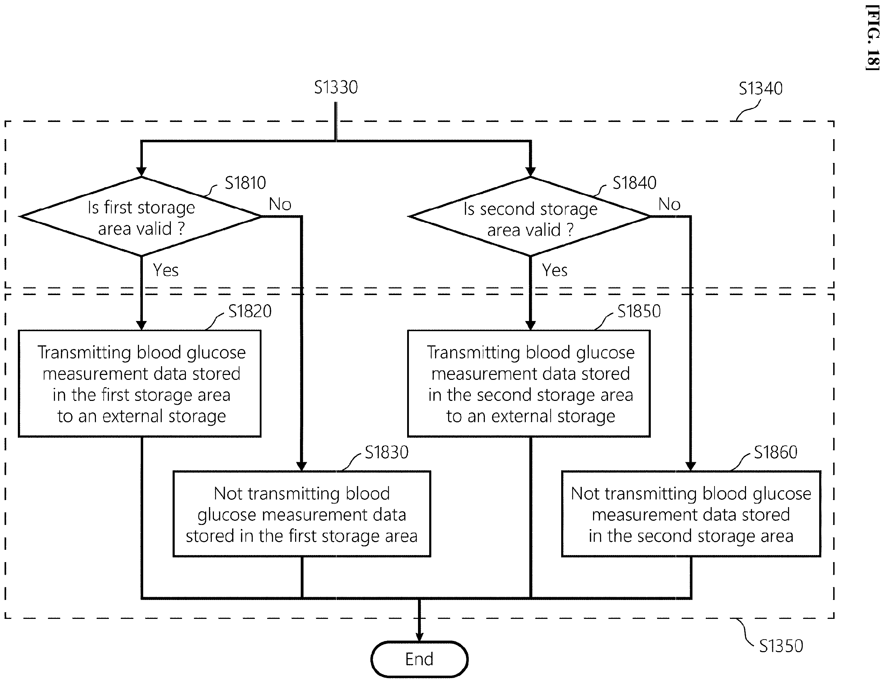

FIG. 18 is an embodiment showing steps S1340 and S1350 of FIG. 13 in detail. Referring to FIG. 18, first, the control unit 120 may check the validity of the blood glucose measurement data stored in the first storage area (S1810).

If the blood glucose measurement data stored in the first storage area is determined to be valid, the communication unit 140 may transmit the blood glucose measurement data stored in the first storage area to an external device (S1820). That is, the control unit 120 may control the communication unit 140 to transmit the blood glucose measurement data stored in the first storage area to the external device.

On the other hand, if the blood glucose measurement data stored in the first storage area is determined to be invalid, the communication unit 140 may not transmit the blood glucose measurement data stored in the first storage area to the external device (S1830). That is, the control unit 120 may control the communication unit 140 not to transmit the blood glucose measurement data stored in the first storage area to the external device.

The control unit 120 may check the validity of the blood glucose measurement data stored in the second storage area (S1840).

If the blood glucose measurement data stored in the second storage area is determined to be valid, the communication unit 140 may transmit the blood glucose measurement data stored in the second storage area to the external device (S1850). That is, the control unit 120 may control the communication unit 140 to transmit the blood glucose measurement data stored in the second storage area to the external device.

On the other hand, if the blood glucose measurement data stored in the second storage area is determined to be invalid, the communication unit 140 may not transmit the blood glucose measurement data stored in the second storage area to the external device (S1860). That is, the control unit 120 may control the communication unit 140 not to transmit the blood glucose measurement data stored in the second storage area to the external device.

Although the above description focuses on the embodiments, these are merely examples and do not limit the present invention. Any person who has an ordinary knowledge in the field to which the present invention pertains will recognize that various modifications and applications not exemplified above are possible within the scope not departing from the essential characteristics of the present invention. For example, each component specifically shown in the embodiment can be modified and implemented. In addition, the differences related to such modifications and applications should be interpreted as being included in the scope of the present invention defined in the appended claims.

### [Description of symbols]

10: Blood glucose measurement device
30: User terminal
110: Measurement unit
120: Control unit
130: Storage unit
140: Communication unit

## Claims

1. A blood glucose measurement device comprising:
a measurement unit that is partially inserted into the body of a subject and generates a blood glucose measurement signal by measuring a blood glucose concentration of the subject;
a control unit that generates blood glucose measurement data by converting the blood glucose measurement signal into digital data;
a storage unit that includes a plurality of storage areas and stores the blood glucose measurement data in the plurality of storage areas; and
a communication unit that transmits the blood glucose measurement data stored in at least one of the plurality of storage areas to an external device,
wherein the control unit determines the validity of the blood glucose measurement data stored in the plurality of storage areas, and controls the communication unit to transmit the blood glucose measurement data stored in at least one of the plurality of storage areas to the external device, based on a result of the determination.

2. The blood glucose measurement device according to claim 1,
wherein the storage unit stores the blood glucose measurement data in a first storage area included in the plurality of storage areas, and
stores the blood glucose measurement data in a second storage area included in the plurality of storage areas,
wherein the blood glucose measurement data stored in the first storage area match with the blood glucose measurement data stored in the second storage area.

3. The blood glucose measurement device according to claim 2,
wherein the communication unit transmits the blood glucose measurement data stored in the first storage area to the external device if the blood glucose measurement data stored in the first storage area is determined to be valid; and
transmits the blood glucose measurement data stored in the second storage area to the external device if the blood glucose measurement data stored in the first storage area is determined to be invalid.

4. The blood glucose measurement device according to claim 1,
wherein the storage unit stores the blood glucose measurement data in the first storage area included in the plurality of storage areas; and
stores the blood glucose measurement data in the second storage area included in the plurality of storage areas if a preset error event is determined to have occurred in the first storage area.

5. The blood glucose measurement device according to claim 1,
wherein the storage unit stores the blood glucose measurement data by crossing the first storage area and the second storage area included in the plurality of storage areas.

6. The blood glucose measurement device according to claim 5,
wherein the storage unit stores first blood glucose measurement data in a first storage space of the first storage area; and
stores second blood glucose measurement data in a second storage space of the second storage area,
wherein the first storage space and the second storage space of the first storage area correspond to the first storage space and the second storage space of the second storage area, respectively, and
the first storage space and the second storage space are continuous with each other.

7. The blood glucose measurement device according to claim 6,
wherein the storage unit stores the first blood glucose measurement data in the first storage space of the first storage area; and
stores the second blood glucose measurement data in the first storage space of the second storage area,
wherein the first storage space of the first storage area corresponds to the first storage space of the second storage area.

8. The blood glucose measurement device according to claim 6 or 7,
wherein the control unit checks the validity of the blood glucose measurement data stored in the first storage area and the second storage area; and
the communication unit includes a step of transmitting blood glucose measurement data determined to be valid among the blood glucose measurement data stored in the first storage area and the second storage area to the external device.

9. A method for managing blood glucose measurement data using a blood glucose measurement device that continuously measures blood glucose of a subject,
the method comprising the steps of: generating a blood glucose measurement signal by measuring blood glucose of the subject;
converting the blood glucose measurement signal into blood glucose measurement data that is a digital signal;
storing the blood glucose measurement data in a plurality of storage areas; determining the validity of the blood glucose measurement data stored in the plurality of storage areas; and
transmitting the blood glucose measurement data stored in at least one of the plurality of storage areas to an external device, based on a result of the determination.

10. The method for managing blood glucose measurement data according to claim 9, wherein the step of storing the blood glucose measurement data includes the steps of:
storing the blood glucose measurement data in a first storage area included in the plurality of storage areas; and
storing the blood glucose measurement data in a second storage area included in the plurality of storage areas,
wherein the blood glucose measurement data stored in the first storage area matches with the blood glucose measurement data stored in the second storage area.

11. The method for managing blood glucose measurement data according to claim 10, wherein the step of transmitting the blood glucose measurement data stored in at least one of the plurality of storage areas to the external device includes the steps of:
transmitting the blood glucose measurement data stored in the first storage area to the external device, if the blood glucose measurement data stored in the first storage area is determined to be valid; and
transmitting the blood glucose measurement data stored in the second storage area to the external device, if the blood glucose measurement data stored in the first storage area is determined to be invalid.

12. The method for managing blood glucose measurement data according to claim 9, wherein the step of storing the blood glucose measurement data includes the steps of:
storing the blood glucose measurement data in a first storage area included in the plurality of storage areas;
determining whether a preset error event has occurred in the first storage area; and
storing the blood glucose measurement data in a second storage area included in the plurality of storage areas, if it is determined that the error event has occurred.

13. The method for managing blood glucose measurement data according to claim 9, wherein the step of storing the blood glucose measurement data stores the blood glucose measurement data by crossing the first storage area and the second storage area included in the plurality of storage areas.

14. The method for managing blood glucose measurement data according to claim 13, wherein the step of storing the blood glucose measurement data includes the step of:
storing first blood glucose measurement data in a first storage space of the first storage area; and
storing second blood glucose measurement data in a second storage space of the second storage area,
wherein the first storage space and the second storage space of the first storage area correspond to the first storage space and the second storage space of the second storage area, respectively, and
the first storage space and the second storage space are continuous with each other.

15. The method for managing blood glucose measurement data according to claim 13, wherein the step of storing the blood glucose measurement data includes the steps of:
storing the first blood glucose measurement data in the first storage space of the first storage area; and
storing the second blood glucose measurement data in the first storage space of the second storage area,
wherein the first storage space of the first storage area corresponds to the first storage space of the second storage area.

16. The method for managing blood glucose measurement data according to claim 14
or 15,
wherein the step of transmitting the blood glucose measurement data stored in at least one of the plurality of storage areas to the external device includes the steps of:
checking the validity of the blood glucose measurement data stored in the first storage area and the second storage area; and
transmitting blood glucose measurement data determined to be valid among the blood glucose measurement data stored in the first storage area and the second storage area to the external device.
